# EUROPÄISCHE PATENTSCHRIFT

(11) **EP 2 572 684 B1**
(45) Veröffentlichungstag und Bekanntmachung des Hinweises auf die Patenterteilung: **04.05.2016**
(21) Anmeldenummer: 12180874.5
(22) Anmeldetag: 17.08.2012
(51) Int. Cl.: A61F 2/95

(54) **Freigabevorrichtung zum Lösen eines medizinischen Implantats von einem Katheter sowie Katheter mit einer Freigabevorrichtung**
Release device for disengaging a medical implant from a catheter and catheter having a release device
Dispositif de libération pour libérer un implant médical d'un cathéter ainsi que cathéter avec dispositif de libération

(30) Priorität: 23.09.2011 US 201161538157 P
(43) Veröffentlichungstag der Anmeldung: 27.03.2013
(73) Patentinhaber: Biotronik AG, 8180 Bülach (CH)
(72) Erfinder: Fargahi, Amir, 8180 Bülach (CH)
(74) Vertreter: Galander, Marcus

(56) Entgegenhaltungen:
- EP-A2- 1 964 532
- US-A1- 2005 080 476
- US-A1- 2007 118 201
- US-A1- 2007 168 014

## Beschreibung

Die Erfindung betrifft eine Freigabevorrichtung zum Lösen eines medizinischen Implantats von einem Katheter sowie einen Katheter mit einer Freigabevorrichtung zum Freigeben eines medizinischen Implantats zur Implantation in einen tierischen und/oder menschlichen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate sind zum Einführen in den Körper mit Kathetern verbunden und müssen am Einsatzort genau platziert und definiert freigegeben werden können. Hierzu ist beispielsweise bekannt, das Implantat durch eine Schiebbewegung freizugeben.

US 2007/0118201 offenbart ein Einführsystem für eine medizinische Vorrichtung wie beispielsweise ein Stent.

Der Erfindung liegt die Aufgabe zugrunde, eine Freigabevorrichtung anzugeben, mit der ein hochpräzises und gezieltes Freigeben eines Implantats erfolgen kann.

Eine weitere Aufgabe ist in der Bereitstellung einer entsprechenden Einführvorrichtung zu sehen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende eine Zahnstange mit einem ersten Geschwindigkeitsbereich und einem zweiten Geschwindigkeitsbereich vorgesehen ist, und wobei die Zahnstange zur Erzeugung einer gezielten Relativbewegung zwischen dem ersten und dem zweiten Einführelement der Einführvorrichtung vorgesehen ist.

Vorteilhaft kann ein einfacher Wechsel von schnelles auf langsames Freigeben erfolgen und umgekehrt. Hierzu sind Mechanismen für schnelles auf langsames Freigeben vorgesehen. Die Erfindung ermöglicht eine einfache Handhabung, insbesondere ein Umschalten zwischen den Geschwindigkeitsbereichen. Dies erlaubt eine unkomplizierte, einfache und schnelle Geschwindigkeitsregulierung bei der Freigabe des Implantats. Die Freigabe des Implantats wird präziser und schneller.

Gemäß einer vorteilhaften Ausgestaltung kann der erste und der zweite Geschwindigkeitsbereich jeweils entlang einer Längserstreckung der Zahnstange angeordnet sein.

Der erste und der zweite Geschwindigkeitsbereich sind an gegenüberliegenden Bereichen der Zahnstange angeordnet. Dies erlaubt eine kompakte und einfache Konstruktion der Freigabevorrichtung.

Gemäß einer vorteilhaften Ausgestaltung kann dem jeweiligen Geschwindigkeitsbereich je ein Wirkelement zugeordnet sein, das mit dem jeweiligen Geschwindigkeitsbereich zusammenwirkt, um die Relativbewegung zwischen dem ersten und dem zweiten Einführelement der Einführvorrichtung zu bewirken. Insbesondere kann der erste und der zweite Geschwindigkeitsbereich eine Zahnreihe sein, wobei die Geschwindigkeitsbereiche voneinander unterschiedliche Zahnabstände aufweisen. Vorteilhaft kann das Wirkelement ein Zahnrad umfassen. Bei einem Zahnradantrieb steht die Drehachse des Zahnrades orthogonal zur Verschieberichtung der Zahnstange. Dies erlaubt auf einfache Weise eine Umsetzung von zwei unterschiedlichen Geschwindigkeitsbereichen der Freigabevorrichtung. Der Verfahrensweg eines Zahnstangenantriebs berechnet sich nach dem mittleren Umfang des Zahnkranzes des antreibenden Zahnrades und dessen Drehzahl. Eine hohe Präzision bei der Positionierung des Implantats kann durch den Einsatz der Zahnräder erreicht werden, statt wie im Stand der Technik ein Schieben und Zurückziehen der Einführelemente einzusetzen. Es ergibt sich stattdessen eine stabile Konstruktion mit zwei Achsen.

Gemäß einer vorteilhaften Ausgestaltung kann das Wirkelement gegenüber einem Gehäuse fixiert sein, das wenigstens um die Zahnstange angeordnet ist. Das Gehäuse kann insbesondere einen Handgriff der Einfrühvorrichtung bilden. Vorzugsweise kann das Wirkelement mit einem Betätigungselement gekoppelt ist, die aus dem Gehäuse ragt. Dies ermöglicht eine leichte und kontrollierte Bedienbarkeit der Freigabevorrichtung.

Gemäß einer vorteilhaften Ausgestaltung kann die Zahnstange eine Durchführung für eines der Einführelemente aufweisen. Dies erlaubt eine kompakte Anordnung, welche das durchgeführte Einführelement stabilisiert und schützt. Ist die Einführvorrichtung ein Katheter, so kann das betreffende Einführelement ein Innenschaft des Katheters sein.

Gemäß einem weiteren Aspekt der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine Freigabevorrichtung zum Lösen des medizinisches Implantats, umfassend einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende eine Zahnstange mit einem ersten Geschwindigkeitsbereich und einem zweiten Geschwindigkeitsbereich vorgesehen ist, wobei die Zahnstange zur Erzeugung einer gezielten Relativbewegung zwischen dem ersten und dem zweiten Einführelement der Einführvorrichtung vorgesehen ist.

Die Einführvorrichtung kann günstigerweise ein Katheter sein Besonders vorteilhaft kann die Einführvorrichtung zur Montage und Freigabe einer Prothese, einer Herzklappe oder eines Stents eingesetzt werden.

Gemäß einer vorteilhaften Ausgestaltung kann ein inneres Einführelement durch die Zahnstange geführt sein. Dies erlaubt eine kompakte Bauweise der Einführvorrichtung.

Gemäß einer vorteilhaften Ausgestaltung kann die Zahnstange an der Schleusenvorrichtung fixiert sein. Die Anordnung kann sehr stabil ausgeführt sein.

Die Erfindung ist nachfolgend beispielhaft, anhand von in Zeichnungen dargestellten Ausführungsbeispielen, näher erläutert. Es zeigen in schematischer Darstellung:
- Fig. 1: einen Schnitt durch ein günstiges Ausführungsbeispiel einer Einführvorrichtung und einer Freigabevorrichtung; und
- Fig. 2: eine Draufsicht auf die Einführvorrichtung und Freigabevorrichtung aus Fig.,1.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

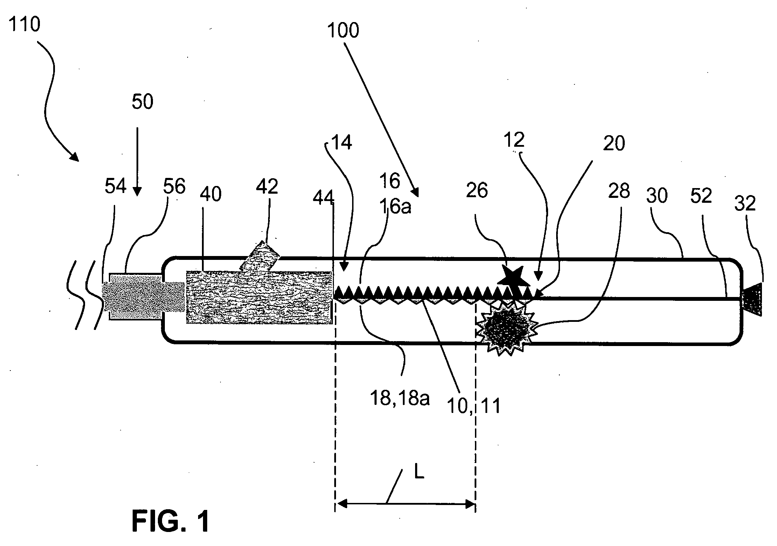

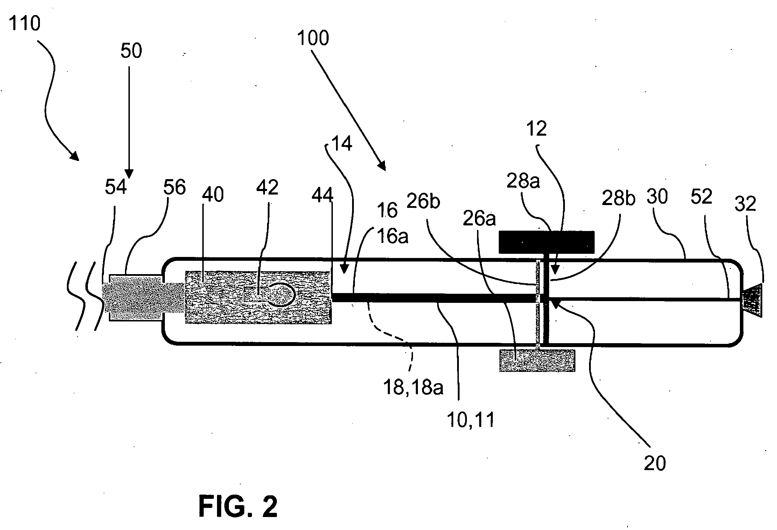

Figur 1 zeigt einen Längsschnitt durch ein günstiges Ausführungsbeispiel einer Freigabevorrichtung 100 einer nur teilweise dargestellten Einführvorrichtung 110. Die Freigabevorrichtung 100 dient zum Lösen eines medizinisches Implantats (nicht dargestellt) von der Einführvorrichtung 110, wobei das Implantat durch eine Relativbewegung zwischen einem ersten und einem zweiten Einführelemente 52, 54 freisetzbar ist. Figur 2 zeigt eine Draufsicht davon.

Die Einführvorrichtung 110 ist beispielsweise ein Katheter mit einem Schaftbereich 50 mit einem Innenschaft 52 und einem diesen umgebenden Außenschaft 54, welcher wiederum von einer Außenhülle 56 umgeben sein kann. Die Einführvorrichtung ist nur an ihrem proximalen Ende dargestellt, das einem Anwender zugewandt ist. Das Implantat (nicht dargestellt) ist üblicherweise am distalen Ende des Schaftbereichs 50 zwischen Innenschaft und Außenschaft platziert und soll am Implantationsort im tierischen oder menschlichen Körper freigegeben werden.

Die Freigabevorrichtung umfasst einen Körper 10 mit einem proximalen Ende 12, das im Benutzungszustand dem Anwender zugewandt ist und einem distalen Ende 14, das im Benutzungszustand vom Anwender entfernt ist. Zwischen dem proximalen und dem distalen Ende 12, 14 ist eine Zahnstange 11 mit einem ersten Geschwindigkeitsbereich 16 und einem zweiten Geschwindigkeitsbereich 18 vorgesehen. Die Zahnstange 11 dient zur Erzeugung einer gezielten Relativbewegung zwischen dem ersten und dem zweiten Einführelement 52, 54 der Einführvorrichtung, die Geschwindigkeitsbereiche 16, 18 sind als Zahnreihen 16a, 18a mit unterschiedlichen Zahnabständen ausgebildet, die jeweils entlang einer Längserstreckung L auf gegenüberliegenden Seiten der Zahnstange 11 angeordnet sind.

Dem jeweiligen Geschwindigkeitsbereich 16, 18 ist je ein Wirkelement 26, 28 in Form eines Zahnrads zugeordnet, das mit der Zahnung des jeweiligen Geschwindigkeitsbereich 16, 18 zusammenwirkt, um die Relativbewegung zwischen dem ersten und dem zweiten Einführelement 52, 54 zum Freigeben des Implantats von der Einführvorrichtung 110 zu bewirken. Dabei weist der erste Geschwindigkeitsbereich 16 eine Zahnreihe 16a mit einer engen Zahnung auf, was eine langsame Bewegung beim Drehen des Zahnrads 26 bewirkt. Der zweite Geschwindigkeitsbereich 18 weist eine Zahnreihe 18a mit weiteren Zahnabständen auf, was eine schnelle Bewegung beim Drehen des Zahnrads 28 bewirkt.

Die beiden Wirkelemente 26, 28 sind gegenüber einem Gehäuse 30, das die Zahnstange 11 bzw. den Körper 10 umgibt, so fixiert, das beim Drehen der Zahnräder die Zahnstange über das eine Einführelement 52, also z.B. den Innenschaft des Katheters, gleitet. Hierzu weist die Zahnstange 11 eine Durchführung 20 das Einführelement 52 auf.

Der Verfahrweg s der Zahnstange 11 ergibt sich aus dem mittleren Umfang U des Zahnkranzes (U = π x d; mit d = mittlerer Durchmesser) des antreibenden Zahnrads 26 oder 28 und der Drehzahl n des Zahnrads 26 oder 28 mit s = U x n.

Das Lumen des Einführelements 52 (Innenschaft) kann mit einem Entlüftungsventil 32 (auch bekannt als Luer-Lock) entlüftet und/oder gespült werden.

Die Zahnstange 11 ist an ihrem proximalen Ende 14 an einer Schleusenvorrichtung 40 an einer Fixierung 44 fixiert. Die Schleusenvorrichtung 40 kann ein so genannter T-Körper sein. Die Schleusenvorrichtung 40 weist ein Entlüftungsventil 42 auf (auch bekannt als Luer-Lock), durch das Luft aus dem Lumen der Einführelemente 52, 54 bzw. aus Zwischenraum zwischen diesen entfernt werden kann und z.B. eine Flüssigkeit eingeführt werden kann. Die Schleusenvorrichtung 40 ist mit einem äußeren der Einführelemente 54 verbunden, also z.B. dem Außenschaft des Katheters, und gegebenenfalls mit einer Schutzhülle 56 um das Einführelement 54.

Die Wirkelemente 26, 28 sind durch aus dem Gehäuse 30 ragende Betätigungselemente 26a, 28b gekoppelt, so dass das jeweilige Zahnrad 26, 28 bequem von außen betätigt werden kann.

Die Betätigungselemente 26a, 28a sind je mit einer Welle 26b, 28b mit dem jeweiligen Wirkelement 26, 28 verbunden, so dass die Achse der Wirkelemente 26, 28 senkrecht zur Bewegungsrichtung der Zahnstange 11 steht.

Die Betätigungselemente 26a und 28a erlauben ein schnelles Wechseln von einer langsamen Bewegung (mit Betätigungselement 26a) zu einer schnellen Bewegung (Betätigungselement 28a) und umgekehrt, indem von dem einen Betätigungselement 26a oder 28a auf das andere 28a oder 26a zugegriffen wird und damit das jeweilige Zahnrad (Wirkelement 26 oder Wirkelement 28) gedreht oder gestoppt wird.

## Patentansprüche

1. Freigabevorrichtung (100) zum Lösen eines medizinisches Implantats von einer Einführvorrichtung (110), bei welcher das Implantat durch eine Relativbewegung zwischen einem ersten und einem zweiten Einführelement (52, 54) freisetzbar ist, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) eine Zahnstange (11) mit einem ersten Geschwindigkeitsbereich (16) und einem zweiten Geschwindigkeitsbereich (18) vorgesehen ist, wobei die Zahnstange (11) zur Erzeugung einer gezielten Relativbewegung zwischen dem ersten und dem zweiten Einführelement (52, 54) der Einführvorrichtung (110) vorgesehen ist, **dadurch gekennzeichnet, dass**
der erste und der zweite Geschwindigkeitsbereich (16, 18) an gegenüberliegenden Bereichen der Zahnstange (11) angeordnet sind

2. Freigabevorrichtung nach Anspruch 1, wobei der erste und der zweite Geschwindigkeitsbereich (16, 18) jeweils entlang einer Längserstreckung (L) der Zahnstange (11) angeordnet sind.

3. Freigabevorrichtung nach einem der vorhergehenden Ansprüche, wobei dem jeweiligen Geschwindigkeitsbereich (16, 18) je ein Wirkelement (26, 28) zugeordnet ist, das mit dem jeweiligen Geschwindigkeitsbereich (16, 18) zusammenwirkt, um die Relativbewegung zwischen dem ersten und dem zweiten Einführelement (52, 54) der Einführvorrichtung (110) zu bewirken.

4. Freigabevorrichtung nach einem der vorhergehenden Ansprüche, wobei der erste und der zweite Geschwindigkeitsbereich (16, 18) eine Zahnreihe (16a, 18b) ist, wobei die Geschwindigkeitsbereiche (16, 18) voneinander unterschiedliche Zahnabstände aufweisen.

5. Freigabevorrichtung nach einem der vorhergehenden Ansprüche, wobei das Wirkelement (26, 28) gegenüber einem Gehäuse (30) fixiert ist, das wenigstens um die Zahnstange (11) angeordnet ist.

6. Freigabevorrichtung nach Anspruch 5, wobei das Wirkelement (26, 28) mit einem Betätigungselement (26a, 28b) gekoppelt ist, die aus dem Gehäuse (30) ragt.

7. Freigabevorrichtung nach einem der vorhergehenden Ansprüche, wobei die Zahnstange (11) eine Durchführung (20) für eines der Einführelemente (52) aufweist.

8. Freigabevorrichtung nach einem der vorhergehenden Ansprüche, wobei das Wirkelement (26, 28) ein Zahnrad umfasst.

9. Freigabevorrichtung nach einem der Ansprüche 5 bis 8, wobei das innere Einführelement (52) ein Entlüftungsventil (32) aufweist.

10. Einführvorrichtung (110) zum Einführen eines medizinischen Implantats, welches durch eine Relativbewegung zwischen einem ersten und einem zweiten Einführelement (26, 28) freisetzbar ist, umfassend eine Freigabevorrichtung (100) zum Lösen des medizinisches Implantats nach einem der vorhergehenden Ansprüche, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) eine Zahnstange (11) mit einem ersten Geschwindigkeitsbereich (16) und einem zweiten Geschwindigkeitsbereich (18) vorgesehen ist, wobei die Zahnstange (11) zur Erzeugung einer gezielten Relativbewegung zwischen dem ersten und dem zweiten Einführelement (52, 54) der Einführvorrichtung (110) vorgesehen ist.

11. Einführvorrichtung nach Anspruch 10, wobei am distalen Ende (12) eine Schleusenvorrichtung (40) angeordnet ist, die mit einem äußeren der Einführelemente (54) verbunden ist.

12. Einführvorrichtung nach Anspruch 10 oder 11, wobei ein inneres Einführelement (52) durch die Zahnstange (11) geführt ist.

13. Einführvorrichtung nach einem der Ansprüche 11 bis 12, wobei die Zahnstange (11) an der Schleusenvorrichtung (40) fixiert ist.

14. Einführvorrichtung nach einem der Ansprüche 10 bis 13, wobei dem jeweiligen Geschwindigkeitsbereich (16, 18) je ein Wirkelement (26, 28) zugeordnet ist, das mit dem jeweiligen Geschwindigkeitsbereich (16, 18) zusammenwirkt, um die Relativbewegung zwischen dem ersten und dem zweiten Einführelement (52, 54) der Einführvorrichtung (110) zu bewirken.

## Claims

1. A release device (100) for disengaging a medical implant from an insertion device (110), in which the implant is releasable by a relative movement between a first insertion element and a second insertion element (52, 54), comprising a body (10) having a proximal end (12), which faces towards a user in the usage state, and a distal end (14), which is remote from the user in the usage state, wherein a toothed rack (11) having a first speed range (16) and a second speed range (18) is provided between the proximal end and the distal end (12, 14), wherein the toothed rack (11) is provided to generate a targeted relative movement between the first insertion element and the second insertion element (52, 54) of the insertion device (110), **characterised in that**
the first speed range and the second speed range (16, 18) are situated on opposing regions of the toothed rack (11).

2. The release device according to Claim 1, wherein the first and the second speed range (16, 18) are each situated along a longitudinal extension (L) of the toothed rack (11).

3. The release device according to one of the preceding claims, wherein an action element (26, 28) is assigned in each case to the respective speed range (16, 18) and cooperates with the respective speed range (16, 18) in order to cause the relative movement between the first insertion element and the second insertion element (52, 54) of the insertion device (10).

4. The release device according to one of the preceding claims, wherein the first speed range and the second speed range (16, 18) are rows of teeth (16a, 18b), wherein the speed ranges (16, 18) have different tooth intervals from one another.

5. The release device according to one of the preceding claims, wherein the action element (26, 28) is fixed in relation to a housing (30), which is situated at least around the toothed rack (11).

6. The release device according to Claim 5, wherein the action element (26, 28) is coupled to an actuation element (26a, 28b), which protrudes out of the housing (30).

7. The release device according to one of the preceding claims, wherein the toothed rack (11) has a feedthrough (20) for one of the insertion elements (52).

8. The release device according to one of the preceding claims, wherein the action element (26, 28) has a gearwheel.

9. The release device according to one of Claims 5 to 8, wherein the inner insertion element (52) has a deaeration valve (32).

10. An insertion device (110) for inserting a medical implant, which is releasable by a relative movement between a first insertion element and a second insertion element (26, 28), comprising a release device (100) for disengaging the medical implant according to one of the preceding claims, comprising a body (10) having a proximal end (12), which faces towards a user in the usage state, and a distal end (14), which is remote from the user in the usage state, wherein a toothed rack (11) having a first speed range (16) and a second speed range (18) is provided between the proximal end and the distal end (12, 14), wherein the toothed rack (11) is provided to generate a targeted relative movement between the first insertion element and the second insertion element (52, 54) of the insertion device (110).

11. The insertion device according to Claim 10, wherein an airlock device (40), which is connected to an outer of the insertion elements (54), is situated at the distal end (12).

12. The insertion device according to Claim 10 or 11, wherein an inner insertion element (52) is guided by the toothed rack (11).

13. The insertion device according to one of Claims 11 to 12, wherein the toothed rack (11) is fixed on the airlock device (40).

14. The insertion device according to one of Claims 10 to 13, wherein an action element (26, 28) is assigned in each case to the respective speed range (16, 18) and cooperates with the respective speed range (16, 18) in order to cause the relative movement between the first insertion element and the second insertion element (52, 54) of the insertion device (110).

## Revendications

1. Dispositif de libération (100) pour enlever un implant médical d'un dispositif d'introduction (110), dans lequel l'implant peut être libéré par un mouvement relatif entre un premier et un deuxième éléments d'introduction (52, 54), comprenant un corps (10) avec une extrémité proximale (12), qui est orientée face à un utilisateur dans l'état d'utilisation, et avec une extrémité distale (14), qui est éloignée de l'utilisateur dans l'état d'utilisation, dans lequel une crémaillère (11) avec un premier domaine de vitesse (16) et un deuxième domaine de vitesse (18) est prévue entre les extrémités proximale et distale (12, 14), dans lequel la crémaillère (11) est prévue pour la création d'un mouvement relatif ciblé entre les premier et deuxième éléments d'introduction (52, 54) du dispositif d'introduction (110), **caractérisé en ce que** les premier et deuxième domaines de vitesse (16, 18) sont agencés sur des domaines de la crémaillère (11) situés l'un en face de l'autre.

2. Dispositif de libération selon la revendication 1, dans lequel les premier et deuxième domaines de vitesse (16, 18) sont agencés chaque fois le long d'une extension longitudinale (L) de la crémaillère (11).

3. Dispositif de libération selon l'une des revendications précédentes, dans lequel un élément actif (26, 28) est associé à chaque domaine de vitesse (16, 18), qui agit conjointement avec le domaine de vitesse (16, 18) correspondant afin de provoquer le mouvement relatif entre les premier et deuxième éléments d'introduction (52, 54) du dispositif d'introduction (110).

4. Dispositif de libération selon l'une des revendications précédentes, dans lequel les premier et deuxième domaines de vitesse (16, 18) sont constitués d'une rangée de dents (16a, 18b), où les domaines de vitesse (16, 18) présentent des espaces entre les dents différents l'un de l'autre.

5. Dispositif de libération selon l'une des revendications précédentes, dans lequel l'élément actif (26, 28) est fixé en face d'un boîtier (30) qui est disposé au moins autour de la crémaillère (11).

6. Dispositif de libération selon la revendication 5, dans lequel l'élément actif (26, 28) est couplé avec un élément d'actionnement (26a, 28b) qui dépasse du boitier (30).

7. Dispositif de libération selon l'une des revendications précédentes, dans lequel la crémaillère (11) présente un passage (20) pour l'un des éléments d'introduction (52).

8. Dispositif de libération selon l'une des revendications précédentes, dans lequel l'élément actif (26, 28) comprend un engrenage.

9. Dispositif de libération selon l'une des revendications 5 à 8, dans lequel l'élément d'introduction interne (52) présente une soupape de mise à l'air (32).

10. Dispositif d'introduction (110) pour l'introduction d'un implant médical, lequel peut être libéré par un mouvement relatif entre un premier et un deuxième éléments d'introduction (26, 28), comprenant un dispositif de libération (100) pour l'enlèvement de l'implant médical, selon l'une des revendications précédentes, comprenant un corps (10) avec une extrémité proximale (12) qui est orientée vers un utilisateur dans l'état d'utilisation et une extrémité distale (14) qui est éloignée de l'utilisateur dans l'état d'utilisation, dans lequel une crémaillère (11) avec un premier domaine de vitesse (16) et un deuxième domaine de vitesse (18) est prévue entre les extrémités proximale et distale (12, 14), dans lequel, pour la création d'un mouvement relatif ciblé, la crémaillère (11) est prévue entre les premier et deuxième éléments d'introduction (52, 54) du dispositif d'introduction (110).

11. Dispositif d'introduction selon la revendication 10, dans lequel, un dispositif de verrouillage (40) est agencé à l'extrémité distale (12), qui est relié avec l'un des éléments d'introduction externe (54).

12. Dispositif d'introduction selon les revendications 10 ou 11, dans lequel un élément d'introduction interne (52) est guidé par la crémaillère (11).

13. Dispositif d'introduction selon l'une des revendications 11 à 12, dans lequel la crémaillère (11) est fixée sur le dispositif de verrouillage (40).

14. Dispositif d'introduction selon l'une des revendications 10 à 13, dans lequel, chaque fois, un élément actif (26, 28) est associé à un domaine de vitesse respectif (16, 18), qui agit conjointement avec le domaine de vitesse (16, 18) correspondant, afin de provoquer le mouvement relatif entre les premier et deuxième éléments d'introduction (52, 54) du dispositif d'introduction (110).
